# EUROPEAN PATENT SPECIFICATION

(11) **EP 1 655 037 B1**
(45) Date of publication and mention of the grant of the patent: **05.08.2015**
(21) Application number: 04746153.8
(22) Date of filing: 15.06.2004
(51) Int. Cl.: A61P 3/06, A61K 31/40, A61K 31/216, A61K 31/505, A61K 31/7052

(54) **SERUM CHOLESTEROL LOWERING AGENT OR PREVENTIVE OR THERAPEUTIC AGENT FOR THE TREATMENT OF ATHEROSCLEROSIS**
MITTEL ZUR SENKUNG DES SERUMCHOLESTERINSPIEGELS ODER MITTEL ZUR PRÄVENTION ODER BEHANDLUNG VON ATHEROSKLEROSE
AGENT VISANT A FAIRE BAISSER LE CHOLESTEROL SERIQUE OU AGENT DE PREVENTION OU THERAPEUTIQUE POUR L'ATHEROSCLEROSE

(30) Priority: 27.06.2003 JP 2003185171
(43) Date of publication of application: 10.05.2006
(73) Proprietor: Kotobuki Pharmaceutical Co., Ltd., Hanishina-gun, Nagano 389-0697 (JP)
(72) Inventor: TOMIYAMA, Hiroshi, Nagano 389-0601 (JP); YOKOTA, Masayuki, Chikuma-shi, Nagano 387-0023 (JP); KOSAKAI, Kazuhiro, Ueda-shi, Nagano 386-1321 (JP)
(74) Representative: Adam, Holger
(86) International application number: PCT/JP2004/008678
(87) International publication number: WO 2005/000353

(56) References cited:
- EP-A1- 1 362 855
- WO-A1-02/50090
- WO-A1-94/14433
- WO-A1-02/066464
- WO-A2-02/058732
- JP-A- 8 501 110
- JP-A- 8 505 141
- JP-A- 9 510 970
- JP-A- 10 512 592
- US-A- 5 756 470
- SUDHOP T ET AL: "CHOLESTEROL ABSORPTION INHIBITORS FOR THE TREATMENT OF HYPERCHOLESTEROLAEMIA" DRUGS, ADIS INTERNATIONAL LTD, NZ, vol. 62, no. 16, 1 January 2002 (2002-01-01), pages 2333-2347, XP008011717 ISSN: 0012-6667
- VAN HEEK MARGARET ET AL: "Comparison of the activity and disposition of the novel cholesterol absorption inhibitor, SCH58235, and its glucuronide, SCH60663" BRITISH JOURNAL OF PHARMACOLOGY, vol. 129, no. 8, April 2000 (2000-04), pages 1748-1754, XP002573180 ISSN: 0007-1188
- WIERZBICKI A S: "NEW LIPID-LOWERING AGENTS" EXPERT OPINION ON EMERGING DRUGS, ASHLEY PUBLICATIONS, GB, vol. 8, no. 2, 1 January 2003 (2003-01-01) , pages 365-376, XP009036380 ISSN: 1472-8214

## Description

### FIELD OF THE INVENTION

The present invention relates to medicinal compositions that are useful as serum cholesterol lowering agent or preventive or therapeutic agent for atherosclerosis, in more detail, it relates to medicinal compositions of a β-lactam cholesterol absorption inhibitor containing a C-glycoside in the molecule combined with cholesterol biosynthesis inhibitors and/or fibrate-type cholesterol lowering agents.

### BACKGROUND OF THE INVENTION

Conventionally, cholesterol biosynthesis inhibitors or fibrate-type cholesterol lowering agents have been widely used for serum cholesterol reduction and prevention or therapy of atherosclerosis, and the combination of β-lactam cholesterol absorption inhibitors and cholesterol biosynthesis inhibitors has been proposed (JP 8-505141). The present applicant has previously published that β-lactam cholesterol absorption inhibitors containing C-glycoside in the molecules have an excellent cholesterol lowering action, and are useful as serum cholesterol lowering agents (WO-02/066464 A1).

WO 94/14433 A1 relates to a combination of a cholesterol biosynthesis inhibitor and a beta-lactam cholesterol absorption inhibitor and describes methods of reducing plasma cholesterol levels and treating or preventing atherosclerosis comprising administering an effective amount of a combination of a cholesterol biosynthesis inhibitor and a beta-lactam cholesterol absorption inhibitor, as well as pharmaceutical compositions and kits useful in those methods.

Sudhop et al (Drugs, Adis International Ltd, NZ, Vol. 62, No. 16, January 2002, pages 2333-2347) describe cholesterol absorption inhibitors for the treatment of hypercholesterolaemia.

EP 1 362 855 A1 relates to beta-lactam compounds, a process for reproducing the same and serum cholesterol-lowering agents containing the same and describes compounds having the following general formula (I) wherein: A₁, A₃ and A₄ are hydrogen atom, halogen atom, alkyl group having one to five carbon atoms, alkoxy group having one to five carbon atoms, -COOR₁, a following formula (b) (wherein: R₁ is hydrogen atom or alkyl group having one to five carbon atoms) or a following formula (a) [wherein: R₂ is -CH₂OH group, -CH₂OC(O)-R₁ group or -CO₂-R₁ group; R₃ is -OH group or -OC(O)-R₁ group; R₄ is -(CH₂)ₖR₅(CH₂)₁- (k and 1 are 0 or 1 more integer; k+1 is 10 or fewer integer); R₅ means bond (single bond (-), -CH=CH-, -OCH₂-, carbonyl group or -CH(OH)-]; one of A₁, A₃ and A₄ in formula (I) is must be the group in above mentioned formula (a); A₂ is alkyl chain having one to five carbon atoms, alkoxy chain having one to five carbon atoms, alkenyl chain having one to five carbon atoms, hydroxyalkyl chain having one to five carbon atoms or carbonylalkyl chain having one to five carbon atoms; n, p, q or r are 0, 1 or 2; or their pharmaceutical acceptable salts.

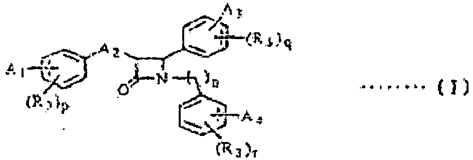

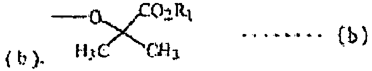

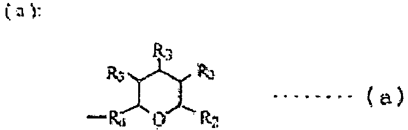

The purpose of the present invention is to supply a more excellent serum cholesterol lowering agent or preventive or therapeutic agent for atherosclerosis.

### DISCLOSURE OF THE INVENTION

The present invention provides a serum cholesterol lowering agent or preventive or therapeutic agent for atherosclerosis consisting of the combination of a compound represented by the following chemical formula or a pharmaceutically acceptable salt thereof and a cholesterol biosynthesis inhibitor and/or a fibrate-type cholesterol lowering agent, wherein said cholesterol biosynthesis inhibitor is at least one HGM CoA reductase inhibitor selected from the group consisting of atorvastatin and rosuvastatin, and said fibrate-type cholesterol lowering agent is fenofibrate.

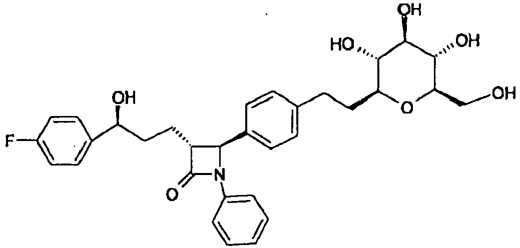

In a preferred embodiment, a container containing the compound represented by the above chemical formula or a pharmaceutically acceptable salt thereof and a container containing a cholesterol biosynthesis inhibitor and/or a fibrate-type cholesterol lowering agent as defined above form a kit by single packaging.

Also, the present invention provides a serum cholesterol lowering agent or preventive or therapeutic agent for atherosclerosis consisting of the mixture of the compound represented by the above chemical formula or pharmaceutical acceptable salts thereof and cholesterol biosynthesis inhibitors and/or fibrate-type cholesterol lowering agents as defined above. Also, the present invention provides a serum cholesterol lowering agent or preventive or therapeutic agent for atherosclerosis forming a kit by single packaging a container containing a compound represented by the above chemical formula or pharmaceutical acceptable salts thereof and a container containing cholesterol biosynthesis inhibitor and/or fibrate-type cholesterol lowering agents as defined above. Also, it is possible to administer a compound represented by the above chemical formula or pharmaceutical acceptable salts thereof and cholesterol biosynthesis inhibitors and/or fibrate-type cholesterol lowering agents as defined above simultaneously or consecutively.

### PREFERRED EMBODIMENT OF THE INVENTION

The present invention provides a serum cholesterol lowering agent or preventive or therapeutic agent for atherosclerosis as defined above. Concretely, this combined medicine means : ① the medicine combined a compound represented by the above chemical formula or pharmaceutical acceptable salts thereof with cholesterol biosynthesis inhibitors as defined above, ② the medicine combined of a compound represented by the above chemical formula or pharmaceutical acceptable salts thereof with fibrate-type cholesterol lowering agents as defined above, ③ the medicine combined of a compound represented by the above chemical formula or pharmaceutical acceptable salts thereof with cholesterol biosynthesis inhibitors and fibrate-type cholesterol lowering agents as defined above. This combined usage means combined administration, and it is possible to administer simultaneously or consecutively.

The compound represented by the above chemical formula or pharmaceutical acceptable salts thereof have serum cholesterol lowering actions. This and other similar compounds are shown in WO 02/066464 A1. These β-lactam compounds, which show cholesterol lowering actions and have C-glycoside in the molecules, show synergistic effects when used in combination with cholesterol biosynthesis inhibitors and/or fibrate-type cholesterol lowering agents for serum cholesterol lowering effect or preventive or therapeutic effect for atherosclerosis.

The medicine of the present invention is administered in oral dosage or non-oral dosage form. And, combined usage of a compound represented by the above formula or pharmaceutical acceptable salts thereof and cholesterol biosynthesis inhibitors and/or fibrate-type cholesterol lowering agents as defined above can be carried out in various forms. For example, the compound represented by the above chemical formula or pharmaceutical acceptable salts thereof and cholesterol biosynthesis inhibitors and/or fibrate-type cholesterol lowering agents as defined above are mixed at the predetermined ratio, furthermore, it is possible to form a combination agent with blended additives and excipients according to the request (a powder agent, a tablet, a granule agent, a capsule agent, a liquid agent, a suspended agent, a suppository, an ointment agent, an inhalation agent and others). Additives and excipients are lubricants, binders, collapses, fillers, buffers, emulsifiers, preservatives, anti-oxidants, coloring agents, coating agents, suspending agents and others.

Also, it is possible to form a kit by single packaging a container containing a compound represented by the above chemical formula or pharmaceutical acceptable salts thereof and a container containing cholesterol biosynthesis inhibitors and/or fibrate-type cholesterol lowering agents as defined above. Also, it is possible to administer a compound represented by the above chemical formula or pharmaceutical acceptable salts thereof and cholesterol biosynthesis inhibitors and/or fibrate-type cholesterol lowering agents as defined above simultaneously or consecutively.

The daily dose of the medicine of the present invention is determined by the potency of the compound administered, the weight, age, and condition of the patient and others. Also, the medicine is administered in a single dose or 2~5 divided doses depending oral dosage or non-oral dosage forms. A compound represented by the above chemical formula or pharmaceutical acceptable salts thereof are administered in the amount of 0.1 ~ 100mg/kg (mammalian weight) per day in division. Cholesterol biosynthesis inhibitors as defined above are administered the amount of 1mg ~3g/kg (mammalian weight) per day in division, and HMG-CoA reductase inhibitors as defined above are administered in the amount of 5~100mg/kg (mammalian weight) per day in division. Fibrate-type cholesterol lowering agents are administered in the amount of 1~1000mg/kg (mammalian weight) per day in division.

### EXAMPLE

In the pharmacological experiments of this example, compound No. 56 (called compound 56 in the following) and compound No. 37 (called compound 37 in the following) as shown in the following Table were used. Compound 56 is according to the invention; compound 37 is used for comparison.

| No. | Structure | mp (°C) | [α]_{D}²⁵ / (C, Solv.) |
|---|---|---|---|
| 37 | 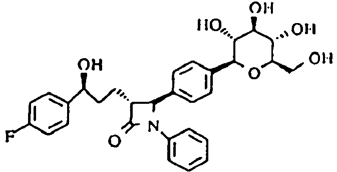 | 80-82 | - |
| 56 | 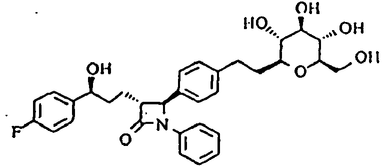 | 82-84 | -49.2 (C=1.0, MeOH) |

### Pharmacological experiment 1

The pharmacological experiment of serum cholesterol lowering action by the combination of compound 56 and atorvastatin or fenofibrate in cholesterol-fed rat.

Male Splague-Dawley rats weighing 300~500g (Nihon SLC Co. Ltd.) were fed MF-2 chow (Nihon Crea Co. Ltd.) until study onset. At the study onset, the chow was changed to MF-2 chow containing 1% cholesterol and 0.5% cholic acid. Compound 56 at 0.3mg/kg, atorvastatin at 1mg/kg or fenofibrate at 10mg/kg dissolved in polyethylene glycol 400 were simultaneously administered once a day for 7 days. Twenty hours after the last administration, blood was collected from the abdominal aorta under ether anaesthesia, and serum was separated. The cholesterol value was measured using Cholesterol E Test Wako (Wako Pure Chemical Co. Ltd.). Furthermore, the effect of combined dosage of compound 56 at 0.3mg/kg and atorvastatin at 1mg/kg or fenofibrate at 10mg/kg were examined similarly. The results are shown in Table 1. The experimental No. of 1~3, 4 and 5 indicates the case of compound 56 alone, atrovastatin alone and fenofibrate alone, respectively. The experimental 5 and 6 indicates the combined dosage examples in the present invention. Each reduction percent is shown as the value to control.

**Table 1**

| Experimental No. | Group | Dose (mg/kg/day) | Number per group | Reduction % of serum cholesterol value |
|---|---|---|---|---|
| 1 | Compound 56 | 0.03 | 6 | 1.9 |
| 2 | Compound 56 | 0.3 | 6 | 6.9 |
| 3 | Compound 56 | 1 | 6 | 33.5 |
| 4 | Atorvastatin | 1 | 6 | 6.2 |
| 5 | Fenofibrate | 10 | 6 | 10.7 |
| 6 | Compound 56 Atorvastatin | 0.3 1 | 6 | 20.2 |
| 7 | Compound 56 Fenofibrate | 0.3 10 | 6 | 41.3 |

From Table 1, in the case of combined dosage compound 56 at 0.3mg/kg/day and atrovastatin 1mg/kg/day (Experimental No. 6), and compound 56 at 0.3mg/kg/day and fenofibrate at 10mg/kg/day (Experimental No. 7), each reduction % of serum cholesterol value was over the sum of reduction % when each agent was administered alone (Experimental No. 2, 4 and 5), indicating synergistic effect.

### Pharmacological experiment 2

Except of the use of compound 37 instead of compound 56, quietly same experiment to pharmacological experiment 1 was carried out. The results are shown in Table 2. Each reduction % is shown as the value to control.

**Table 2**

| Experimental No. | Group | Dose (mg/kg/day) | Number per group | Reduction % of serum cholesterol value |
|---|---|---|---|---|
| 11 | Compound 37 | 0.03 | 6 | 5.6 |
| 12 | Compound 37 | 0.3 | 6 | 18.0 |
| 13 | Compound 37 | 1 | 6 | 31.0 |
| 14 | Atorvastatin | 1 | 6 | 6.2 |
| 15 | Fenofibrate | 10 | 6 | 10.7 |
| 16 | Compound 37 Atorvastatin | 0.3 1 | 6 | 31.5 |
| 17 | Compound 37 Fenofibrate | 0.3 10 | 6 | 39.5 |

From Table 2, in the case of combined dosage compound 37 at 0.3mg/kg/day and atrovastatin 1mg/kg/day (Experimental No. 16), and compound 37 at 0.3mg/kg/day and fenofibrate at 10mg/kg/day (Experimental No. 17), the reduction % of serum cholesterol values were over the sum of reduction % when each agent was administered alone (Experimental No. 12, 14 and 15), indicating synergistic effect.

### INDUSTRIAL APPLICABILITY

The medicine consisting of the combination of a compound represented by the above chemical formula or pharmaceutical acceptable salts thereof and cholesterol biosynthesis inhibitors and/or fibrate-type cholesterol lowering agents as defined above show the synergistic effect and an excellent serum cholesterol lowering effect or preventive or therapeutic effect for atherosclerosis. Therefore, it is useful for serum cholesterolol lowering or preventive or therapy for atherosclerosis.

## Claims

1. A serum cholesterol lowering agent or preventive or therapeutic agent for atherosclerosis consisting of the combination of a compound represented by the following chemical formula or a pharmaceutically acceptable salt thereof and a cholesterol biosynthesis inhibitor and/or a fibrate-type cholesterol lowering agent, wherein said cholesterol biosynthesis inhibitor is at least one HGM CoA reductase inhibitor selected from the group consisting of atorvastatin and rosuvastatin, and said fibrate-type cholesterol lowering agent is fenofibrate.

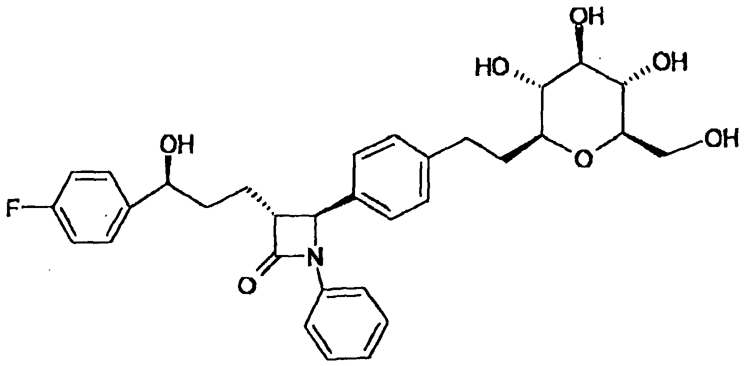

2. The serum cholesterol lowering agent or preventive or therapeutic agent for atherosclerosis of claim 1, wherein a container containing the compound represented by the chemical formula mentioned in claim 1 or a pharmaceutically acceptable salt thereof and a container containing a cholesterol biosynthesis inhibitor and/or a fibrate-type cholesterol lowering agent mentioned in claim 1 form a kit by single packaging.

## Patentansprüche

1. Serumcholesterinsenkendes Mittel oder präventives oder therapeutisches Mittel gegen Atherosklerose, bestehend aus der Kombination einer Verbindung, die durch die folgende chemische Formel dargestellt wird, oder eines pharmazeutisch annehmbaren Salz davon und eines Cholesterinbiosynthese-Inhibitors und/oder eines cholesterinsenkenden Mittels des Fibrattyps, wobei der Cholesterinbiosynthese-Inhibitor wenigstens ein HGM-CoA-Reduktase-Inhibitor ist, der aus der Gruppe, bestehend aus Atorvastatin und Rosuvastatin, ausgewählt ist und das cholesterinsenkende Mittel des Fibrattyps Fenofibrat ist.

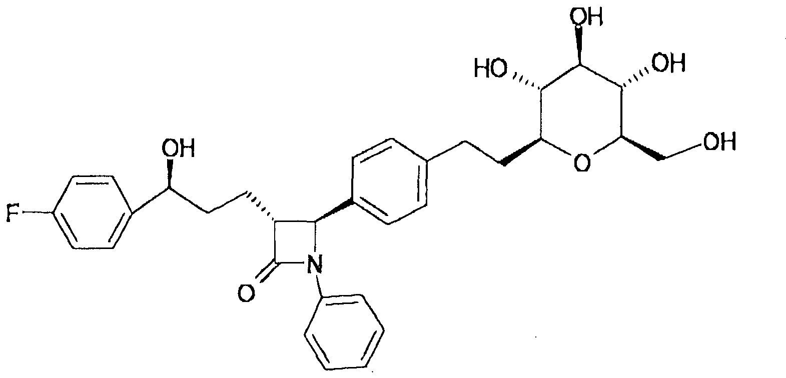

2. Serumcholesterinsenkendes Mittel oder präventives oder therapeutisches Mittel gegen Atherosklerose nach Anspruch 1, wobei ein Behälter, der die Verbindung, die durch die in Anspruch 1 genannte chemische Formel dargestellt wird, oder ein pharmazeutisch annehmbares Salz davon enthält, und ein Behälter, der einen Cholesterinbiosynthese-Inhibitor und/oder ein cholesterinsenkendes Mittel des Fibrattyps, das oben in Anspruch 1 genannt ist, enthält, durch Einzelverpackung einen Kit bilden.

## Revendications

1. Agent hypocholestérolémiant ou agent préventif ou thérapeutique pour l'athérosclérose, constitué de la combinaison d'un composé représenté par la formule chimique suivante ou un sel pharmaceutiquement acceptable de celui-ci et un inhibiteur de biosynthèse de cholestérol et/ou un agent hypocholestérolémiant de type fibrate, ledit inhibiteur de biosynthèse de cholestérol est au moins un inhibiteur de HGM CoA réductase choisi dans le groupe constitué de l'atorvastatine et de la rosuvastatine, et ledit agent hypocholestérolémiant de type fibrate est le fénofibrate.

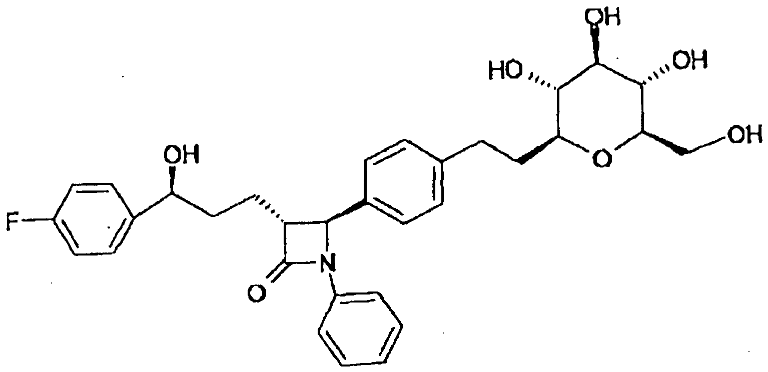

2. Agent hypocholestérolémiant ou agent préventif ou thérapeutique pour l'athérosclérose de la revendication 1, un récipient contenant le composé représenté par la formule chimique mentionnée dans la revendication 1 ou un sel pharmaceutiquement acceptable de celui-ci et un récipient contenant un inhibiteur de biosynthèse de cholestérol et/ou un agent hypocholestérolémiant de type fibrate mentionné dans la revendication 1 formant un kit par emballage unique.
